# DEMANDE DE BREVET EUROPEEN

(11) **EP 0 575 303 A1**
(43) Date de publication de la demande: **22.12.1993**
(21) Numéro de dépôt: 93870088.7
(22) Date de dépôt: 26.05.1993
(51) Int. Cl.: C07C 51/347, C07C 51/367, C07C 69/712, C07C 59/68

(54) **Un procédé pour la préparation de l'acide 5-(2,5-diméthylphénoxy)-2,2-diméthyl pentanoique**

(30) Priorité: 16.06.1992 BE 9200561
(71) Demandeur: S.A. OMNICHEM N.V., B-1435 Mont-St. Guibert (BE)
(72) Inventeur: De Cock, Etienne, B-1853 Grimbergen (BE); Van Brussel, Willy, B-9230 Wetteren (BE); Mangelschots, August, B-9070 Destelbergen (BE)
(74) Mandataire: Van Malderen, Michel

(57) **Abrégé**

Selon l'invention, on réalise d'abord la réaction d'un ester alkyle supérieur de l'acide 2-méthylpropanoïque avec le sel de métal alcalin d'une amine dialkylée pour obtenir un sel de métal alcalin suivie de la condensation avec un 1,3-dihalogéno propane en vue d'obtenir l'intermédiaire de formule III. 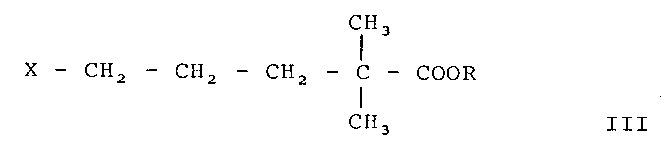
(X représente un atome d'hologène).

Ce composé de formule est condensé avec un sel de métal alcalin de 2,5 diméthylphénol pour donner le sel de métal alcalin de l'acide 5-(2,5-diméthylphénoxy)-2,2-diméthyl pentanoïque qui est ensuite neutralisé.

## Description

La présente invention est relative à un procédé de synthèse d'acides phénoxypentanoïques ainsi qu'à leurs sels et aux produits intermédiaires obtenus dans ce procédé. La présente invention se rapporte, plus précisément, à un nouveau procédé de préparation de l'acide 5-(2,5-diméthylphénoxy)-2,2-diméthyl pentanoïque, plus connu sous le nom générique "Gemfibrozil" de formule I, médicament commercialisé pour ses propriétés hypolipémiantes (P. Samuel, Am. J. Med., Mai 23, 1983, pp. 23-27).

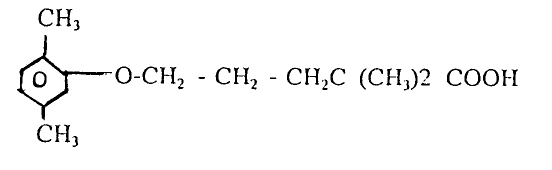

Plusieurs procédés de fabrication du Gemfibrozil ont été décrits dans les brevets suivants :
1) Brevet européen n° EP 0 226 161 qui décrit la condensation du sel de métal alcalin d'un ester alkyle inférieur de l'acide 2-méthyl propanoïque avec le 1-3-dibromo ou 1-chloro-3-bromopropane. L'intermédiaire obtenu est condensé avec le 2,5-diméthylphénol sous forme de sel alcalin suivi de l'hydrolyse immédiate du groupement ester.
2) Brevet allemand DE 4 115 540 qui décrit la condensation d'un sel de métal alcalin d'un polyester de l'acide 2-méthyl propanoïque avec le 1,3-dibromo ou 1-bromo-2-chloropropane. L'intermédiaire obtenu est condensé avec le 2,5-diméthylphénol sous forme de sel alcalin ou d'ester. Le polyester obtenu est alors hydrolysé.

Les procédés décrits ci-dessus comportent des désavantages importants :
a) difficulté de purification des esters d'alkyle inférieur ou des polyesters de l'acide 2-méthylpropanoïque;
b) emploi de solvants polaires diméthylformamide ou diméthylsufoxyde qui, par solubilité complète dans l'eau, sont très difficilement récupérables.

Le nouveau procédé pour l'obtention du composé de formule I implique dans une première étape la réaction d'un ester alkyle supérieur de l'acide 2-méthylpropanoïque avec le sel de métal alcalin d'une amine dialkylée pour obtenir un sel de métal alcalin de formule II : 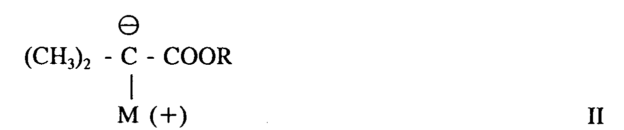
dans laquelle R représente un radical alkyle supérieur et M un métal alcalin, suivie de la condensation avec un 1,3-dihalogéno propane en vue d'obtenir l'intermédiaire de formule III. 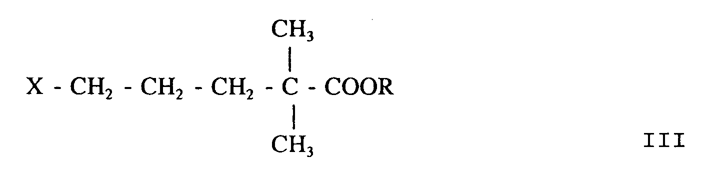
dans laquelle X représente un atome d'halogène.

Dans une seconde étape, le composé de formule III est condensé avec un sel de métal alcalin de 2,5 diméthylphénol pour donner le sel de métal alcalin de l'acide 5-(2,5-diméthylphénoxy)-2,2-diméthyl pentanoïque de formule IV : 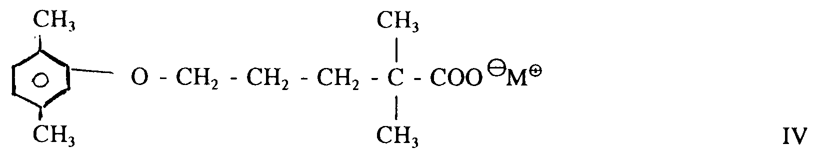
dans lequel M^{⊕} représente un métal alcalin.

Dans une troisième étape, la neutralisation du sel de métal alcalin de formule IV donne le composé de formule I.

Selon le procédé décrit ci-dessus, le composé de formule I est obtenu avec un rendement total supérieur à 80% et selon un procédé industriel plus économique.

De manière plus détaillée, le procédé de l'invention consiste :
a) dans une première étape, à préparer un ester alkyle supérieur de l'acide 5-chloro ou 5-bromo-2,2-diméthylpentanoïque par une C-alkylation d'un ester alkyle supérieur de l'acide 2-méthylpropanoïque avec le 1-bromo-3-chloro propane ou 1,3-dibromo propane. Cette réaction nécessite initialement la formation d'un sel de métal alcalin de l'atome de carbone tertiaire de l'ester isobutyrique suivi par l'alkylation avec un 1,3-dihalogéno propane dans le même réacteur.
   Le terme "ester alkyle supérieur" se rapporte à des radicaux alkyles ou cycloalkyles, contenant entre 5 et 8 atomes de carbone, et, plus précisément, à des radicaux alkyles ou cycloalkyles comme sec-pentyl, isopentyl, cyclopentyl, cyclohexyl, 2-Me (3 ou 4) cyclohexyl, cycloheptyl et cyclooctyl.
   Les produits de départ, plus précisément les esters alkykes supérieurs de l'acide isobutytrique, peuvent être obtenus facilement par des procédés industriels. De plus, ces produits peuvent être facilement distillés et purifiés pour éliminer les résidus d'eau, des alcools alkyles supérieurs et d'acide isobutyrique.
   Des rendements très élevés sont obtenus dans la réaction d'alkylation quand on utilise, de préférence, l'ester cyclohexyle de l'acide 2-méthyl propanoïque et le 1-bromo-3-chloro-propane. Cette alkylation est effectuée de préférence dans un solvant anhydre aprotique tel que le tétrahydofuranne et le diméthoxyéthane ou dans un mélange de solvants anhydres aprotiques tel que le tétrahydrofuranne/ hydrocarbure ou tétrahydrofuranne/hydrocarbure aromatique, plus spécifiquement le tétrahydrofuranne/toluène ou éthylbenzène.
   Le sel de métal alcalin de l'ester alkyle supérieur de l'acide 2-méthyl-propanoïque est obtenu à partir de lithium ou sodium dans un solvant aprotique, d'une dialkyle amine, essentiellement la diisopropylamine, d'un accepteur d'hydrogène tel que le styrène, isoprène ou naphtaline à une température comprise entre 20°C et 60°C, de préférence entre 30 et 40°C, suivie par l'addition de l'ester alkyle supérieur de l'acide 2-méthyl-propionique à une température entre - 20°C et + 20°C de préférence entre - 15 et 5°C.
   Ensuite, le 1,3 di-bromo ou le 1-bromo-3-chloro propane est introduit, dans le même réacteur, goutte à goutte, à une température entre - 20°C et 30°C, de préférence entre - 15 et 0°C. Le milieu réactionnel est ensuite agité pendant une à 15 heures, de préférence entre 3 à 4 heures jusqu'à réaction complète.
   L'isolement de l'ester alkyle supérieur de l'acide 5-chloro-2,2-diméthyl pentanoïque du mélange réactionnel est réalisé par distillation du solvant de réaction, par l'addition d'eau et par l'extraction par un solvant organique, tel que l'hexane, le cyclohexane, le toluène ou l'éthylbenzène. Les phases organiques sont lavées à l'eau jusqu'à neutralité, filtrées et concentrées sous vide. Le produit brut est obtenu avec un rendement quasi quantitatif et peut être utilisé comme tel dans la réaction suivante.
b) Dans une deuxième étape, à préparer un sel de métal alcalin tel que le sodium ou le potassium de l'acide 5-(2,5-diméthyl-phénoxy)2,2-diméthyl pentanoïque par une O-alkylation du 2,5-diméthyl phénol avec l'ester alkyle supérieur de l'acide 5-chloro- ou 5-bromo-2,2-diméthyl pentanoïque en présence d'une solution concentrée d'une base alcaline forte, telle que l'hydroxyde de sodium ou de potassium et d'un catalyseur de transfert de phase, tel que les sels d'ammonium quaternaires, plus précisément le tétrabutylammonium chloride, bromide ou bisulfate.
   L'alkylation peut être réalisée sous atmosphère inerte, préférablement azote ou argon à des températures comprises entre 100° et 150°C, de préférence entre 110° et 120°C; le temps de réaction peut varier entre 5 et 20 heures suivant les conditions et le catalyseur employé.
   L'hydrolyse de l'ester alkyle supérieur est effectué, dans le même réacteur, par l'addition d'un solvant aprotique apolaire, tel que le cyclohexane, le toluène, ou l'éthylbenzène et d'une base alcaline, telle que l'hydroxyde de sodium ou de potassium, de préférence l'hydroxyde de sodium solide. La température de l'hydrolyse est comprise entre 100° et 120°C; le temps de réaction peut varier entre 5 et 20 heures suivant les conditions employées.
   Après réaction complète, le sel de métal alcalin de l'acide 5-(2,5-diméthylphénoxy)-2,2-diméthylpentanoïque est isolé du milieu réactionnel par l'addition d'eau et décantation de la phase aqueuse à une température comprise entre 50°C et 100°C, de préférence 70°C. La phase aqueuse est lavée avec un solvant aprotique apolaire, tel que le toluène et ensuite refroidie pour laisser cristalliser le sel alcalin du Gemfibrozil, qui est isolé par filtration et lavé à l'eau.
c) Dans une troisième étape, on obtient le Gemfibrozil pur par acidification avec un acide fort d'une solution de sel de métal alcalin du Gemfibrozil dans l'eau ou un mélange eau/solvant organique polaire, tel que les alcools, les cétones ou les éthers cycliques, à une température comprise entre 20°C et 100°C, de préférence entre 30°et 50°C.

Les exemples suivants illustrent de manière non limitative le procédé conduisant au produit

### Exemple 1 : Préparation de l'ester cyclohexyle de l'acide 5-chloro-2.2-diméthyl pentanoïque

Dans un ballon de 2000 ml, équipé d'un agitateur mécanique, d'un thermomètre, d'une arrivée d'argon et d'un réfrigérant, on place 232,7 g (2,3 moles) de diisopropylamine anhydre, 300 ml d'hexane anhydre, 800 ml de tétrahydrofuranne anhydre et 16,0 g (2,3 moles) de lithium en granulés. Ensuite, on introduit progressivement 119,8 g (1,15 mole) de styrène dilué dans 150 ml de tétrahydrofuranne de façon à maintenir la température du milieu réactionnel entre 37-40°C. En fin d'addition du styrène, on maintient le milieu réactionnel à cette température pendant 4 à 5 heures.

Le mélange réactionnel est ensuite refroidi à - 15°C et on ajoute 340,6 g (2 moles) d'isobutyrate de cyclohexyle pendant deux heures de façon à garder la température du milieu réactionnel en dessous de - 12°C. Après agitation pendant une heure à - 15°C, on ajoute 314,9 g (2 moles) de 1-bromo-3-chloro propane pendant 3 à 4 heures de façon à garder la température en dessous de - 12°C.

En fin d'addition, on maintient le milieu à - 10°C jusqu'à réaction complète (5 heures). On chauffe progressivement le milieu à 80°C et on distille le mélange de solvants organiques. Ensuite, on introduit 1000 ml de toluène et 330 ml d'eau et on acidifie par une solution d'acide chlorhydrique concentré (115 ml).

On sépare la phase aqueuse et on lave la phase organique deux fois par 130 ml d'eau. La phase organique est ensuite distillée à sec sous vide (rendement quantitatif : pureté GC : > 95 %) .

### Example 2 : Préparation du sel de sodium de l'acide 5-(2,5-diméthylphénoxy)-2,2-diméthyl pentanoïque

Dans un ballon de 1000 ml, équipé d'un agitateur mécanique, d'un thermomètre, d'une arrivée d'argon et d'un bain chauffant, on place 160 ml d'eau, 46 g (1,15 mole) d'hydroxyde de sodium, 122,2 g (1 mole) de 2,5-diméthylphénol, 6,4 g (0,02 mole) de bromure de tétrabutyl ammonium et 259,7 g (1 mole) d'ester cyclohexyle de l'acide 5-chloro-2,2-diméthyl pentanoïque (pureté 95%). Sous atmosphère inerte d'argon et sous agitation, on chauffe le milieu réactionnel à 110-115°C pendant 10 heures jusqu'à réaction complète.

Ensuite, on ajoute 80 g (2 moles) d'hydroxyde de sodium solide et 500 ml de toluène et on chauffe à 108 - 112°C pendant 4 à 6 heures jusqu'à réaction complète. Le mélange réactionnel est versé sur de l'eau (2 l) et les deux phases sont séparées à une température entre 70 et 80°C.

La phase aqueuse est acidifiée par de l'acide chlorhydrique (environ 1 mole) jusqu'à pH 9.4 et lavée deux fois avec du toluène à une température entre 70 et 80°C. La phase aqueuse est ensuite refroidie sous agitation et on laisse cristalliser lentement le sel sodique du Gemfibrozil. On obtient le produit par filtration à 0°C et par lavage avec de l'eau glacée. Rendement : 90%.

### Exemple 3 : Préparation de l'acide 5-(2,5-diméthylphénoxy)-2,2-diméthyl pentanoïque (Gemfibrozil)

Dans un réacteur de 3000 ml, équipé d'un agitateur mécanique, d'un thermomètre, d'une arrivée d'argon et d'un réfrigérant, on place 850 ml d'eau et 91 ml (1,1 mole) d'acide chlorhydrique concentré. Sous agitation forte et à une température comprise entre 20 et 25°C, on ajoute, goutte à goutte, une solution aqueuse chaude du sel sodique de Gemfibrozil, (0,9 mole) dans 1250 ml d'eau. Le produit précipité est isolé par filtration, à 20°C, lavé à l'eau et séché sous vide à une température entre 45 et 50°C. Rendement 95%.

## Revendications

1. Un procédé de préparation de l'acide 5-(2,5-diméthyl phénoxy)-2,2-diméthyl pentanoïque qui consiste :
a) dans une première étape, à faire réagir un ester alkyle supérieur de l'acide 2-méthyl-propanoïque avec un sel de métal alcalin d'une amine disubstituée dans un solvant anhydre aprotique polaire pour obtenir un sel de métal alcalin de l'atome de carbone tertiaire représenté par la formule : 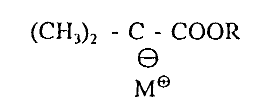 dans laquelle R est un radical alkyle supérieur et M est un métal alcalin et ensuite avec un 1,3-dihalogéno propane pour obtenir un intermédiaire représenté par la formule :
X- CH₂ - CH₂ - CH₂ (CH₃)₂ COOR
dans laquelle X est un atome de chlore ou de brome et R est un radical alkyle supérieur.
b) dans une deuxième étape, à faire réagir l'intermédiaire mentionné ci-dessus avec un sel alcalin de 2,5-diméthylphénol pour obtenir l'ester alkyle supérieur de l'acide 5-(2,5-diméthylphénoxy)-2,2-diméthyl pentanoïque et ensuite à saponifier l'ester alkyle supérieur en question dans le même réacteur pour ainsi pouvoir isoler le sel de métal alcalin de l'acide 5-(2,5-diméthoxyphénoxy)-2,2-diméthyl pentanoïque représenté par la formule : 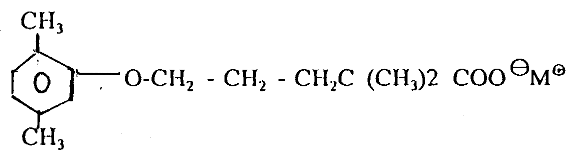 dans laquelle M représente un métal alcalin.
c) dans une troisième étape, à neutraliser le sel de métal alcalin de l'acide mentionné ci-dessus pour obtenir le Gemfibrozil pur.

2. Procédé, selon la revendication 1, caractérisé en ce que l'ester alkyle supérieur de l'acide 2-méthyl propanoïque est l'isobutyrate de cyclohexyle.

3. Procédé, selon la revendication 1, caractérisé en ce que, pour l'étape a), on utilise le 1-bromo-3-chloro-propane, l'amine disubstituée est la diisopropylamine, le métal alcalin est le lithium et le solvant est le tétrahydrofuranne.

4. Procédé, selon la revendication 1, caractérisé en ce que, pour la réaction b), on utilise l'ester cyclohexyle de l'acide 5-chloro-2-diméthyl pentanoïque sous sa forme brute.

5. Procédé, selon la revendication 1, caractérisé en ce que on utilise, pour la réaction b), une base alcaline et un catalyseur de transfert de phase à une température comprise entre 110-120°C.

6. Procédé, selon la revendication 5, caractérisé en ce qu'on utilise une solution d'hydroxyde de sodium concentrée et le bromure de tétrabutylammonium.

7. Procédé, selon la revendication 1, caractérisé en ce que, pour la réaction b), l'hydrolyse de l'ester cyclohexyle est effectué dans le même réacteur à une température élevée dans un solvant organique aprotique apolaire avec une base alcaline.

8. Procédé, selon la revendication 7, caractérisé en ce que le solvant est le toluène, la base alcaline est l'hydroxyde de sodium et la température est comprise entre 110 et 120°C.

9. Procédé, selon la revendication 1, caractérisé en ce que la purification de l'étape b) est effectuée par lavage d'une solution aqueuse du sel alcalin du Gemfibrozil à pH contrôlé par un solvant aprotique apolaire à une température élevée suivi par cristallisation du sel de métal alcalin dans un mélange eau/solvant organique polaire.

10. Procédé, selon la revendication 9, caractérisé en ce que le sel alcalin est le sel de sodium, le pH de lavage est compris entre 9 et 10, le solvant de lavage est le toluène, la température est comprise entre 70 et 80°C et le solvant de cristallisation est l'eau.

11. Procédé, selon la revendication 1, caractérisé en ce que, pour l'étape décrite en c), on acidifie une solution du sel alcalin dans un mélange eau/solvant polaire avec un acide fort à une température basse.

12. Procédé, selon la revendication 11, caractérisé en que le sel alcalin est le sel de sodium, le solvant est l'eau, l'acide est l'acide chlorhydrique et la température est comprise entre 20-25°C.
